Europäisches Patentamt

European Patent Office

Office européen des brevets

(19)

(11) **EP 0 603 029 B1**

(12) **FASCICULE DE BREVET EUROPEEN**

(45) Date de publication et mention de la délivrance du brevet:
**11.09.1996 Bulletin 1996/37**

(51) Int Cl.6: **G01N 3/32**, G01N 33/42, G01N 3/24

(21) Numéro de dépôt: **93402965.3**

(22) Date de dépôt: **08.12.1993**

(54) **Appareil et procédé d'essai mécanique permettant d'étudier la fissuration en fatigue de matériaux routiers**

Verfahren und Vorrichtung zur Prüfung des Ermüdungsrisswachstums von Strassendeckenmaterialien

Device and method for the mechanical testing of fatigue crack growth of road materials

(84) Etats contractants désignés:
**AT BE CH DE DK ES FR GB IE IT LI MC NL SE**

(30) Priorité: **08.12.1992 FR 9214973**

(43) Date de publication de la demande:
**22.06.1994 Bulletin 1994/25**

(73) Titulaire: **COLAS S.A.**
**F-92653 Boulogne-Billancourt Cédex (FR)**

(72) Inventeurs:
- **Fuentes, Joseph**
  **F-19300 Egletons (FR)**
- **Caperaa, Serge**
  **F-19300 Egletons (FR)**
- **Petit, Christophe**
  **F-19300 Egletons (FR)**
- **Michaut, Jean-Paul**
  **F-91190 Gif Sur Yvette (FR)**

(74) Mandataire: **Michelet, Alain et al**
**Cabinet Harlé et Phelip**
**21 rue de la Rochefoucauld**
**75009 Paris (FR)**

(56) Documents cités:
FR-A- 1 473 309          US-A- 1 789 846
US-A- 2 754 681

- database Inspec Acces.Nu. 3221842 Juin 88 & Exp. Mechanics vol 28 n.2 pages 146-153 Swartz et al. 'Model-II fracture parameter estimates for concrete from beam specimens'
- DATABASE WPI Section EI, Week 9144, 18 Décembre 1991 Derwent Publications Ltd., London, GB; Class S, AN 91323666/44 & SU-A-1 613 925 (MOSC ATOMENERGOPROE) 15 Décembre 1990
- DATABASE WPI Section EI, Week 9143, 11 Décembre 1991 Derwent Publications Ltd., London, GB; Class S, AN 91316816-43 & SU-A-1 629 806 (KHARK) 23 Février 1991
- MATERIAUX ET CONSTRUCTIONS vol. 14, no. 79 , Janvier 1981 , FR pages 25 - 33 BUTLER ET AL. 'preliminary data using a flexural cyclaic loading machine'
- STRAIN vol. 18, no. 4 , Novembre 1982 , NEWCASTLE-UPON-TYNE GB pages 149 - 159 BUTLER 'assesing the deterioration of plain and fibrous concrete'
- JOURNAL OF TESTING AND EVALUATION vol. 17, no. 3 , 1989 , PHILADELPHIA US pages 157 - 165 MOR ET AL. 'fatigue of submerged concrete'

## Description

L'invention concerne un appareil et un procédé d'essai mécanique permettant d'étudier la fissuration en fatigue de matériaux routiers.

Dans le domaine de la construction routière, le dimensionnement des différentes couches d'un revêtement d'une route doit être fait, entre autre, au vu du phénomène de fissuration qui résulte d'une fatigue des matériaux utilisés. Sauf dans le cas d'une fissure accidentelle, les fissures commencent à se former, sur la face inférieure d'une couche et se propagent vers la face supérieure de cette couche avec une vitesse qui est fonction de plusieurs paramètres caractéristiques du champ mécanique autour de la pointe de sollicitation. Ces fissures ne peuvent être dépistées pratiquement qu'au moment où elles apparaissent à la surface de la route. On essaie donc de se donner des moyens qui permettent de déterminer les intervalles certains pendant lesquels les risques de fissuration sont minimals.

La loi de résistance à la fatigue d'un milieu fissuré relie généralement la vitesse de propagation d'une fissure à un ou plusieurs paramètres caractéristiques du champ mécanique lors d'un chargement répété, généralement cyclique. Ainsi, la loi de Paris, par exemple, relie la propagation par cycle da/dN à la variation du facteur d'intensité de contrainte ($K_{max}$ - $K_{min}$) au cours d'un cycle:

$$da/dN = A (K_{max} - K_{min})^m,$$

où A et m sont deux paramètres intrinsèques du matériau,

a est la longueur de la fissure parallèlement à la force de sollicitation appliquée,
N est le nombre de sollicitations,
$K_{max}$ et $K_{min}$ sont les contraintes, respectivement maximum et minimum résultant des sollicitations.

Le dimensionnement des couches de roulement permettant d'assurer la résistance de la route au phénomène de la fissuration en fatigue, connu aussi sous le vocable de "reflective cracking", repose sur l'intégration de lois de ce type. Le calcul des structures routières et la modélisation numérique permettent de prévoir des valeurs de contrainte sous les actions climatiques et sous l'action du trafic. Cependant, il reste à déterminer par des essais des constantes relatives à la résistance du matériau en fissuration.

Plusieurs appareils d'essai ont été mis au point, ayant la plupart du temps pour objectif de tester l'efficacité des divers procédés "anti-fissures". Les résultats obtenus sont essentiellement qualitatifs et permettent une classification de ces procédés par un critère tel que le nombre de cycles à la rupture.

Le document US-A-1 789 846 décrit une machine de mesure des réponses à des essais de cisaillement. Le document MATERIAUX ET CONSTRUCTION vol. 14, no. 79, Janvier 1981, FR, pages 25-33 BUTLER ETAL•: "Preliminary data using a flexural cyclic loading machine to test plain and fibrous concrete" décrit une machine dans laquelle les éprouvettes sont sollicitées en flexion au moyen de deux broches distancées l'une de l'autre dans le sens de la longueur de l'éprouvette.

L'analyse des contraintes auxquelles une route -est soumise par la circulation, montre qu'à chaque passage d'un véhicule, chacune des roues soumet le lieu de passage successivement

- lors de son approche: à un cisaillement,
- lors de son passage: à une flexion, et
- lors de son éloignement: à un cisaillement.

Il s'ensuit que l'évaluation d'un matériau routier, sur une éprouvette, doit comprendre aussi bien des essais en flexion que des essais en cisaillement. Alors que de nombreux traités théoriques et essais pratiques traitent de la fissuration sollicitée par flexion, le phénomène de la fissuration sollicitée par cisaillement est peu exploré; il est pourtant prépondérant pour des couches de roulement de faible épaisseur.

L'invention a pour but de proposer un appareil et un procédé permettant de simuler les composantes du trafic avec une large plage de variations sollicitant une fissure aussi bien par flexion que par cisaillement et en prenant en compte les propriétés de recouvrance du matériau.

Le but de l'invention est atteint par un appareil d'essai mécanique permettant d'étudier la fissuration en fatigue de matériaux routiers, l'appareil comprenant un bâti comportant un socle muni de logements destinés à recevoir une éprouvette de forme générale allongée, et un portique porteur de moyens de sollicitation de l'éprouvette, les moyens de sollicitation comportant un axe destiné à transmettre à l'éprouvette les efforts de sollicitation perpendiculairement à l'axe longitudinal de l'éprouvette, et comprenant des moyens de mesure des efforts de sollicitation et de la progression d'un front de fissure.

Conformément à l'invention, l'appareil d'essai est caractérisé en ce que l'axe est muni, à son extrémité libre, d'une tête interchangeable formée de façon à pouvoir transformer les efforts de sollicitation exercés par l'axe en des contraintes de cisaillement ou de flexion.

Il s'agit de concevoir un appareil d'essai capable de relier, de manière sûre, les valeurs des contraintes, qui sont des valeurs mesurables et contrôlables, aux caractéristiques de la fissure produite de manière à accéder aux paramètres intrinsèques du matériau, des valeurs qui ne sont pas directement mesurables, tel que le facteur d'intensité de contrainte.

De manière générale, les éprouvettes utilisées dans l'appareil d'essai selon l'invention, sont de forme parallélépipédiques. Cependant, toute autre forme de l'éprouvette est possible. Afin d'obtenir une direction prévisible de la propagation de la fissure, les formes et dimensions doivent être choisies telles que le mode de sollicitation soit unique. Ceci permet la mise en place d'un dispositif de mesure de la propagation du front des fissures.

L'invention concerne également les caractéristiques ci-après, considérées isolément ou selon toutes leurs combinaisons techniquement possibles:

- La tête de l'axe destinée à transmettre à l'éprouvette des efforts de sollicitation comprend un étrier destiné à être monté sur l'éprouvette entre les logements recevant l'éprouvette, l'étrier étant formé de façon à pouvoir exercer un effort de va-et-vient sur l'éprouvette. Cette disposition permet d'assurer et de garantir le contrôle d'une force imposée, notamment pour des fréquences variables et élevées, en tenant compte des fonctions de relaxation du matériau.
- L'étrier enferme l'éprouvette sur une longueur, mesurée dans la direction de l'étendue longitudinale de l'éprouvette, suffisamment grande pour obtenir l'effet de cisaillement par la coopération de l'étrier avec les logements.
- La tête de l'axe est interchangeable. Cette disposition permet d'une part, d'utiliser l'appareil d'essai aussi bien pour des essais de flexion que pour des essais de cisaillement, en choisissant une tête appropriée, et permet d'autre part d'utiliser des éprouvettes de toutes formes.
- L'appareil d'essai comprend des moyens permettant d'agir simultanément, mais de manière indépendante, sur les différents types d'efforts de sollicitation. Cette disposition tient compte des facteurs principaux qui caractérisent le trafic routier, à savoir la fréquence, liée à la vitesse d'un véhicule au type de chaussée et à la position de la fissure, l'amplitude, liée à la charge sur l'essieu, et la période de repos entre le passage de deux essieux, période durant laquelle le phénomène de la recouvrance peut s'exercer.
- L'appareil d'essai comprend des moyens de mesure permettant d'évaluer la réaction de l'éprouvette.
- Le socle du bâti de l'appareil d'essai présente une surface sur laquelle sont disposés des éléments de support dont chacun est muni d'un élément d'appui, chaque ensemble élément de support/élément d'appui étant formé de façon à pouvoir éliminer la transmission vers l'éprouvette d'effets parasitaires provenant d'une sollicitation intempestive du bâti. Cette autonomie relative de la zone d'attache ou de logement de l'éprouvette par rapport au reste de l'appareillage va en même temps dans le sens d'une plus grande liberté de forme et dimensions possibles de l'éprouvette.
- L'appareil d'essai comprend une enceinte climatique destinée à recevoir l'éprouvette pour y effectuer les essais, l'enceinte climatique étant munie d'un passage étanche destiné à l'axe et étant reliée à une armoire climatique. Cette disposition tient compte du fait que la fonction de relaxation du matériau dépend de la température du matériau.
- L'appareil d'essai est relié à un dispositif de commande programmable apte à commander les différents moyens de sollicitation, de climatisation et de mesure selon une séquence d'étapes d'essai prédéterminée.

Le but de l'invention est également atteint par un procédé d'essai mécanique permettant d'étudier la fissuration en fatigue de matériaux routiers.

Conformément à l'invention ce procédé comprend principalement les étapes suivantes:

- former une éprouvette de forme générale allongée en un matériau destiné à être utilisé pour un revêtement d'une route,
- amorcer une fissure sur une partie de la surface de l'éprouvette, la fissure étant orientée sensiblement transversalement par rapport à l'étendue longitudinale de l'éprouvette,
- monter l'éprouvette dans un appareil d'essai selon l'invention,
- munir l'éprouvette d'un capteur de progression du front de fissure,
- relier le capteur au dispositif de mesure,
- fixer l'axe de sollicitation de l'appareil d'essai à l'éprouvette,
- programmer le dispositif de commande avec les différents paramètres d'essai nécessaires au déroulement de l'essai,
- mesurer et enregistrer la progression du front de fissure, et
- déterminer les paramètres intrinsèques du matériau routier sur la base des résultats de mesure enregistrés.

L'invention concerne également les caractéristiques ci-après, considérées isolément ou selon toutes leurs combinaisons techniquement possibles:

- De préférence, l'éprouvette a la forme d'un parallélépipède. Il est cependant concevable que d'autres formes de l'éprouvette soient plus avantageuses, que ce soit au vu du matériau utilisé ou que ce soit dans l'optique de simuler certaines contraintes appliquées sur une couche d'épaisseur non homogène.
- La fissure est amorcée sur l'éprouvette sur le plan destiné à être orienté, lorsque l'éprouvette est montée dans l'appareil d'essai en regard de la surface du socle du bâti, sur laquelle sont disposés les éléments de support. Dans le cas d'une éprouvette ayant une autre forme que celle d'un parallélépipède, l'amorce de la fissure est faite sur une partie de la surface de l'éprouvette, orientée sensiblement transversalement par rapport à l'orientation de l'axe de sollicitation.

- L'éprouvette est formée en un matériau monocouche.
- L'éprouvette est formée en un matériau multicouches.

De manière générale, les essais effectués sur une éprouvette monocouche donnent des résultats plus concluants que ceux effectués sur une éprouvette multicouches. Il est cependant concevable d'effectuer des essais sur une éprouvette multicouches en complément aux essais effectués sur une éprouvette monocouche pour étudier l'influence du type de fonction entre deux couches sur la fissuration de l'ensemble d'un revêtement multicouches.

- Les paramètres intrinsèques d'un matériau routier sont les paramètres A et m de la loi de Paris.

La fissuration provoquée par cisaillement étant, en général, différente de celle provoquée par flexion, il convient de préciser que $A_c$ et $A_f$ désignent, respectivement pour les essais de cisaillement et de flexion, des constantes pour un matériau routier donné et que $m_c$ et $m_f$ désignent, respectivement pour les essais de cisaillement et de flexion, les exposants de la loi de Paris pour un matériau routier donné.

- Le capteur de progression du front de fissure est constitué par au moins une couche de fils de résistance imprimés. Dans le cas d'une éprouvette parallélépipédique, sur chacune des deux faces longitudinales orientées parallèlement à l'axe de sollicitation, un film sur lequel est imprimé un réseau de fils de résistance destinés à se déchirer fil par fil au fur et à mesure de la progression du front de fissure est appliqué. Il est cependant concevable d'utiliser comme capteur de progression, notamment dans le cas d'une éprouvette à surface bombée, une couche d'une peinture de résistance.

L'invention sera décrite plus en détail, en référence aux dessins qui concernent un mode de réalisation donné à titre d'exemple non limitatif. Dans ces dessins:

La Figure 1 montre de façon schématique, la structure de l'appareil d'essai mécanique selon l'invention, et
la Figure 2, un schéma de principe de l'interconnexion de l'appareil d'essai selon la Figure 1 avec ses modules de commande et d'exploitation.

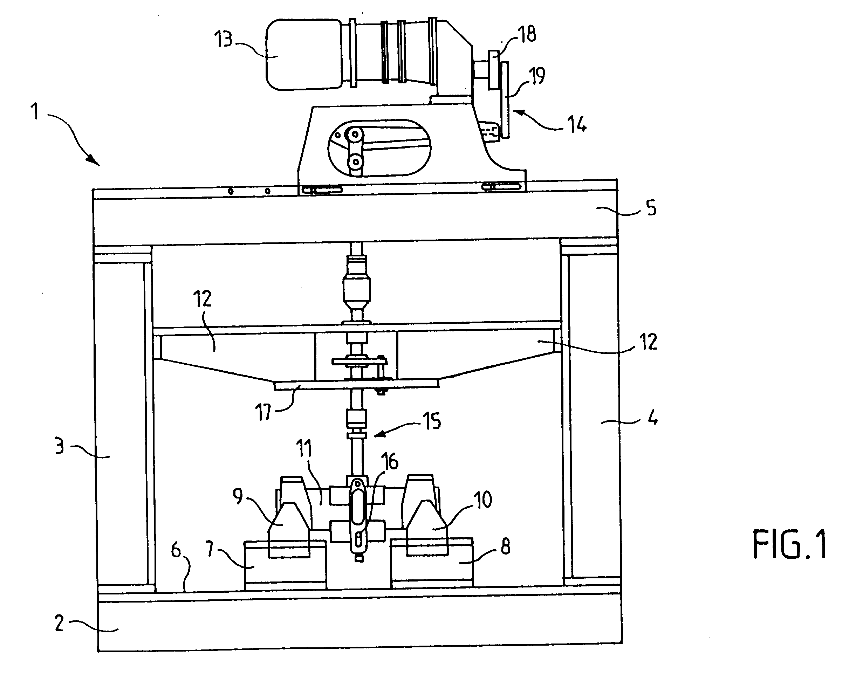

Selon un mode préféré de réalisation, représenté sur la Figure 1, l'appareil d'essai mécanique comprend d'abord un bâti 1, comportant un socle 2 sur lequel sont élevés deux montants 3 et 4, coiffés par un portique 5. Le socle 2 présente entre les deux montants 3 et 4 une surface supérieure horizontale 6 sur laquelle sont disposés deux éléments de support 7 et 8. Chacun des éléments de support 7 et 8 est muni d'un élément d'appui, respectivement 9 et 10. Les éléments d'appui 9 et 10 sont des étriers oscillants réglables sur la fibre neutre de l'éprouvette. Ces deux étriers sont fixés chacun sur un chariot (non représenté) permettant un mouvement horizontal libre. Ils servent de logements à une éprouvette parallélépipédique 11. L'éprouvette 11 a habituellement une longueur de 400 mm, une largeur de 50 mm et une hauteur de 100 mm. La forme et la disposition des éléments de support 7 et 8 et des éléments d'appui 9 et 10 assurent d'une part, la surélévation nécessaire de l'éprouvette 11 par rapport à la surface 6 pour pouvoir soumettre l'éprouvette 11 à des contraintes de flexion et de cisaillement et, d'autre part, une réduction maximale de la transmission de vibrations ou de toute autre sollicitation intempestive provenant du bâti 1.

L'appareil d'essai mécanique comprend, en outre, une traverse 12 montée entre les deux montants 3 et 4 du bâti 1. De plus, un moteur 13 et une transmission 14 sont montés sur le portique 5. La transmission 14 étant reliée mécaniquement au moteur 13, l'effort de sollicitation développé par le moteur 13 passe par la transmission 14 vers un axe de sollicitation 15 muni d'une tête interchangeable 16. L'axe 15 est orienté perpendiculairement par rapport à l'axe longitudinal de l'éprouvette 11.

La tête 16 à la forme d'un étrier destiné à fixer l'axe 15 à l'éprouvette 11. Pour obtenir l'effet de cisaillement, l'étrier 16 est monté sur l'éprouvette 11 entre les deux éléments d'appui 9 et 10. De plus, l'étrier 16 est de forme générale allongée pour pouvoir enfermer l'éprouvette sur une longueur suffisamment grande de la partie centrale.

La longueur de l'étrier 16 est déterminée en fonction de la distance entre les deux éléments d'appui 9 et 10. En général, l'étrier 16 a une longueur d'environ 30 mm. Sa hauteur est d'environ 110 mm et sa largeur d'environ 60 mm. Cet étrier est évidé de chaque côté, dans sa longueur de façon à permettre de suivre visuellement la propagation de la fissure. A l'intérieur de l'étrier 16, deux mors mobiles indépendants (non représentés), placés de part et d'autre de l'encoche réalisée, sont fixés sur l'éprouvette 11 par un cylindre guidé par un ressort et réglé par vis.

L'éprouvette 11 est insérée dans l'étrier 16 goupillé sur un poinçon servant à transmettre l'effort. Le poinçon (non représenté) a un rayon de courbure de 200 mm et est solidaire de l'axe 15.

L'utilisation d'un étrier comme tête de sollicitation permet d'attacher l'axe 15 à l'éprouvette 11 de manière telle que la sollicitation ne porte pas seulement sur la déformation de l'éprouvette (va), mais garantie en outre le retour (vient) de la partie centrale de l'éprouvette à la même position de repos à chaque cycle.

Pour assurer une orientation régulière de l'axe 15, la traverse 12 est munie de moyens de guidage 17. De préférence, ces moyens de guidage 17 sont constitués par un passage cylindrique traversant entièrement la traverse 12 dans une direction sensiblement parallèle à l'étendue des montants 3 et 4.

Pour permettre de faire varier les facteurs essentiels de la simulation du chargement, les réglages suivants sont prévus:

- la fréquence de sollicitation est réglée par la vitesse de rotation du moteur 13;
- les variations d'amplitude de sollicitation sont obtenues par le réglage de la position d'un excentrique 18 et d'un bras de levier 19, autorisant un réglage du déplacement imposé avec une précision de l'ordre du millimètre;
- un programme de pilotage de la commande du moteur prend en compte les durées de travail et de repos voulues par l'utilisateur.

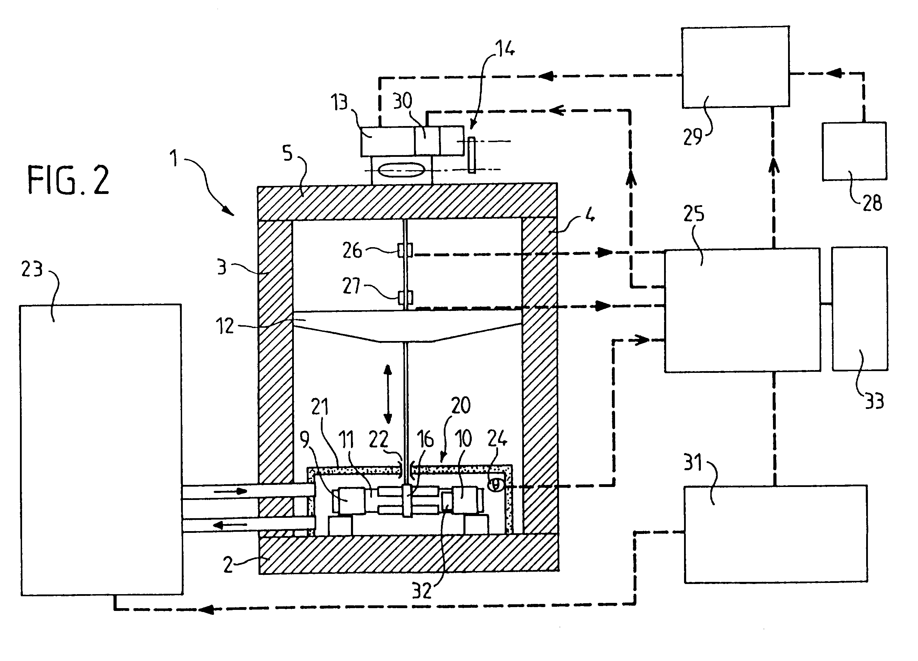

La Figure 2 montre l'implantation de l'appareil d'essai mécanique selon l'invention dans un entourage de modules de commande et d'exploitation. Il est bien entendu que cette présentation très schématique ne montre que les modules et éléments qui sont nécessaires pour la compréhension de l'invention.

Pour tenir compte du fait que la relaxation du matériau routier dépend de la température, l'essai est pratiqué à l'intérieur d'une enceinte, elle-même alimentée à partir d'une armoire climatique. Dans le mode de réalisation préféré de l'invention, une enceinte climatique 20 est disposée sur le socle 2 du bâti 1. L'enceinte climatique 20 enferme l'éprouvette 11 tenu par les éléments d'appui 9 et 10 et présente dans une paroi supérieure 21, un passage 22 climatiquement étanche pour l'axe 15. La climatisation est assurée par une armoire 23 reliée à l'enceinte climatique 20.

Bien qu'il soit concevable d'introduire l'appareil d'essai entier dans une enceinte climatique, il semble préférable de s'en tenir à la version de réalisation décrite ci-avant, parce que la fonction de relaxation du matériau dépend de la température propre du matériau et non pas de la température ambiante. Cette différence est importante dans la mesure où, à l'encontre des situations habituellement rencontrées, la température de l'éprouvette est comparativement indépendante de la température ambiante. Tout au moins, la température de l'éprouvette suit la température ambiante avec un décalage assez important. Ceci est dû au fait que l'effort mécanique appliqué sur l'éprouvette représente un travail de foulage ayant pour conséquence un échauffement de l'éprouvette. En conséquence, il convient de choisir la fréquence et l'amplitude de sollicitation de façon à éviter un échauffement supplémentaire de l'éprouvette.

La température intérieure de l'enceinte 20 est mesurée à l'aide d'un thermomètre 24 qui est relié à une centrale de mesure 25.

Pour mesurer la force et l'amplitude de sollicitation exercées par l'axe 15, la traverse 12 est munie d'un capteur de force 26 et d'un capteur de déplacement 27, tous les deux reliés à la centrale de mesure 25.

Pour pouvoir varier la vitesse de rotation du moteur 13, deux moyens sont prévus. D'une part, l'opérateur de l'appareil d'essai selon l'invention a à sa disposition un pupitre 28. D'autre part, la variation de la vitesse du moteur 13 peut être effectuée à partir de la centrale de mesure 25. Pour cela, la centrale de mesure 25 et le pupitre 28 sont reliés au moteur 13 au moyen d'un circuit d'entrée 29. Le circuit d'entrée 29 peut comprendre, par exemple, un dispositif de priorité, donnant priorité aux signaux de commande venant du pupitre 28 par rapport aux signaux de commande provenant de la centrale de mesure 25.

Dans une variante du mode de réalisation de l'appareil d'essai décrit jusqu'ici, le moteur 13 peut être équipé avec un embrayage-frein 30 qui permet de gérer les temps de travail et de repos de l'appareil. L'embrayage-frein 30 est relié à la centrale de mesure 25.

La manipulation de l'appareil d'essai et la commande des différents modules et éléments nécessaires à son fonctionnement, peut être commandée par un ordinateur qui est désigné sur la Figure 2 par le numéro de référence 31. L'ordinateur 31 peut comprendre notamment des programmes spécifiques pour certains types de sollicitation, correspondant à des circonstances spécifiques de circulation.

L'exploitation des différents résultats d'essais obtenus se fait selon des techniques généralement connues et dont la description détaillée est omise ici. En plus de l'enregistrement numérique ou en courbes des différentes données d'essai, l'observation de la progression du front d'une fissure est effectuée à l'aide d'un capteur 32, disposé sur l'éprouvette 11. Le capteur 32 est relié à la centrale de mesure 25 et à un dispositif d'exploitation 33. De préférence, la progression du front d'une fissure est enregistrée par le dispositif 33 sous forme de tables, ces tables indiquant, par exemple, la progression du front d'une fissure en millimètre par cent mouvements de sollicitation.

**Revendications**

1. Appareil d'essai mécanique permettant d'étudier la fissuration en fatigue de matériaux routiers aussi bien par flexion que par cisaillement, l'appareil comprenant un bâti (1) comportant un socle (2) muni de logements (9, 10) destinés à recevoir une éprouvette (11) de forme générale allongée, un portique (6) porteur de moyens (13 à 15) de sollicitation de l'éprouvette (11), les moyens (13 à 15) de sollicitation comportant un axe (15) destiné à transmettre à l'éprouvette (11) des efforts de sollicitation perpendiculairement à l'axe longitudinal de l'éprouvette (11), et comprenant des moyens de mesure des efforts de sollicitation (26, 27), et des moyens de mesure de la progression d'un front de fissure (32), l'axe (15) étant muni, à son extrémité libre, d'une tête (16) interchangeable formée de façon à pouvoir transformer les efforts de sollicitation exercés par l'axe (15) en des contraintes de cisaillement ou de flexion.

2. Appareil selon la revendication 1, caractérisé en ce que la tête (16) comprend un étrier destiné à être monté sur l'éprouvette (11) entre les logements (9, 10) recevant l'éprouvette (11), l'étrier (16) étant formé de façon à pouvoir exercer un effort bi-directionnel sur l'éprouvette (11).

3. Appareil selon la revendication 2, caractérisé en ce que l'étrier (16) enferme l'éprouvette (11) sur une longueur, mesurée dans la direction de l'étendue longitudinale de l'éprouvette (11) suffisamment grande pour obtenir l'effet de cisaillement par la coopération de l'étrier (16) avec les logements (9, 10).

4. Appareil selon l'une quelconque des revendications 1 à 3, caractérisé en ce qu'il comprend des moyens de variation (25, 28) permettant d'agir simultanément, mais de manière indépendante, sur différents caractères des efforts de sollicitation.

5. Appareil selon l'une quelconque des revendications 1 à 4, caractérisé en ce qu'il comprend des moyens de mesure (32, 33) permettant d'évaluer la réaction de l'éprouvette (11).

6. Appareil selon l'une quelconque des revendications 1 à 5, caractérisé en ce que le socle (2) présente une surface (6) sur laquelle sont disposés des éléments de support (7, 8) dont chacun est muni d'un élément d'appui (9, 10), chaque ensemble élément de support/élément d'appui (7, 9; 8, 10) étant formé de façon à pouvoir éliminer une transmission vers l'éprouvette (11), d'effets parasitaires provenant d'une sollicitation intempestive du bâti (1).

7. Appareil selon l'une quelconque des revendications 1 à 6, caractérisé en ce qu'il comprend une enceinte climatique (20) destinée à recevoir l'éprouvette (11) pour y effectuer les essais, l'enceinte climatique (20) étant munie d'un passage (22) étanche destiné à l'axe (15) et étant reliée à une armoire climatique (23).

8. Appareil selon l'une quelconque des revendications 1 à 7, caractérisé en ce qu'il est relié à un dispositif de commande (31) programmable apte à commander les différents moyens de sollicitation (13 à 15), de climatisation (23) et de mesure (32, 33) selon une séquence d'étapes d'essai prédéterminée.

9. Procédé d'essai mécanique permettant d'étudier la fissuration en fatigue de matériaux routiers aussi bien par flexion que par cisaillement, comprenant les étapes:

- former une éprouvette (11) de forme générale allongée en un matériau destiné à être utilisé pour une couche supérieure d'une route,
- amorcer une fissure sur une partie de la surface de l'éprouvette (11), la fissure étant orientée sensiblement transversalement par rapport à l'étendue longitudinale de l'éprouvette (11),
- monter l'éprouvette (11) dans un appareil d'essai selon l'une quelconque des revendications 1 à 8,
- munir l'éprouvette (11) d'un capteur (32) de progression du front de fissure,
- relier le capteur au dispositif de mesure (26, 27),
- fixer l'axe de sollicitation de l'appareil d'essai à l'éprouvette (11) au moyen d'une tête (16) interchangeable permettant de transformer les effets de sollicitation exercés par l'axe en des contraintes de cisaillement ou de flexion,
- programmer le dispositif de commande avec les différents paramètres d'essai de cisaillement ou de flexion nécessaires au déroulement de l'essai,
- mesurer et enregistrer la progression du front de fissure, et
- déterminer les paramètres intrinsèques du matériau routier sur la base des résultats de mesure enregistrés.

10. Procédé selon la revendication 9, caractérisé en ce que l'éprouvette (11) a la forme d'un parallélépipède et en ce que la fissure est amorcée sur le plan destiné à être orienté, lorsque l'éprouvette (11) est montée dans l'appareil d'essai, en regard de la surface du socle du bâti (1), sur laquelle sont disposés les éléments de support.

11. Procédé selon la revendication 9 ou 10, caractérisé en ce que l'éprouvette (11) est formée en un matériau monocouche.

12. Procédé selon la revendication 9 ou 10, caractérisé en ce que l'éprouvette (11) est formée en un matériau multicouches.

13. Procédé selon l'une des revendications 9 à 12, caractérisé en ce que les paramètres intrinsèques sont les paramètres A et m de la loi de Paris.

14. Procédé selon l'une des revendications 9 à 13, caractérisé en ce que le capteur de progression (26, 27) est constitué par au moins une couche de fils de résistance imprimés.

## Claims

1. A device for the mechanical testing of fatigue crack growth of road materials, whereas the said crack growth may have been caused by flection as well as shearing loads, whereas the device comprises a frame (1) containing a base (2) fitted with recesses (9, 10) designed for accommodating a test-piece (11) of a given length, a frame (6) carrying means (13 to 15) for imparting loads to the test-piece (11), whereas the stressing means (13 to 15) comprise an axle (15) designed for transmitting to the test piece (11), stressing loads perpendicular to the longitudinal axis of the test piece (11) and exhibiting means to measure the stressing loads (26, 27), means to measure the progress of a crack front (32) whereby the axle (15) is fitted, at its free end, with an interchangeable head (16) so that the stressing loads imparted by the axle (15) can be converted into shearing or flection loads.

2. A device according to claim 1, characterised in that the head (16) comprises a clamp designed to be mounted on the test piece (11) between the recesses (9, 10) accommodating the test piece (11), whereas the clamp (16) is formed in order to exert a bidirectional load onto the test piece (11).

3. A device according to claim 2, characterised in that the clamp (16) encloses the test piece (11) over a length, measured in the direction of the longitudinal stretch of the test piece (11) sufficient to obtain the shearing effect by the co-operation of the clamp (16) with the recesses (9, 10).

4. A device according to any of the claims 1 to 3, characterised in that it contains variation means (25, 28) enabling to act simultaneously, but independently, on various components of the loads.

5. A device according to any of the claims 1 to 4, characterised in that it contains measuring means (32, 33) enabling to assess the reaction of the test piece (11).

6. A device according to any of the claims 1 to 5, characterised in that the base (2) exhibits a surface (6) on which are arranged supporting elements (7, 8) each of those being fitted with a bearing element (9 10), whereas each supporting element/bearing element assembly (7, 9; 8, 10) is formed in order to avoid any transmission, towards the test piece (11), of spurious effects caused by untimely loads onto the frame (1).

7. A device according to any of the claims 1 to 6, characterised in that it contains a climatic enclosure (20) designed for accommodating the test piece (11) in order to carry out the tests, whereby the climatic enclosure (20) is fitted with a tight passage (22) designed for the axle (15) and linked to a climatic cabinet (23).

8. A device according to any of the claims 1 to 7, characterised in that it is linked to a programmable control device (31) capable of controlling the various means for imparting the loads (13 to 15), the means for modifying the climatic conditions (23) as well as the measuring means (32, 33) following a pre-set sequence of testing phases.

9. A method for the mechanical testing to study the fatigue crack growth of road materials, a crack growth caused by flection as well as by shearing loads, comprising the following phases :

    - forming a test piece (II), more or less longitudinal in shape, made of a material designed for usage on the upper layer of a road,
    - starting a crack on a section of the surface of the test piece (II), whereas the crack is oriented more or less transversally with respect to the longitudinal stretch of the test piece (II),
    - installing the test piece (II) in a testing device according to any of the claims 1 to 8,
    - fitting the test piece (II) with a sensor (32) to monitor the progress of the crack front,
    - linking the sensor to the measuring device (26, 27),
    - fixing the stressing axis of the testing device to the test piece (II) using an interchangeable head (16) enabling to transform the stressing effects imparted by the axle into shearing or flection stresses,
    - programming the control device with the various parameters to test the shearing or flection loads necessary to the operation of the test,
    - measuring or recording the progress of the crack front, and
    - determining the intrinsic parameters of the road material on the basis of the recorded measuring results.

10. A method according to claim 9, characterised in that the test piece (II) has the form of a parallelepipedon and in that the crack is started on the plane designed to be oriented, when the test piece (II) is mounted in the testing device, opposite the surface of the base of the frame (I), on which are arranged the supporting elements.

11. A method according to claim 9 or 10, characterised in that the test piece (II) is made of a single layer material.

12. A method according to claim 9 or 10, characterised in that the test piece (II) is made of a multilayer material.

13. A method according to one of the claims 9 to 12, characterised in that the intrinsic parameters are the parameters A and m of Paris's law.

14. A method according to one of the claims 9 to 13, characterised in that the progression sensor (26, 27) is made of at least one layer of printed resistance wires.

**Patentansprüche**

1. Vorrichtung zur Prüfung des Ermüdungsrißwachstums von Straßendeckenmaterialien sowohl durch Biegung als auch durch Scherung, wobei die Vorrichtung einen Rahmen (1), der einen Sockel (2) einschließt, der mit Lagern (9,10) versehen ist, die zur Aufnahme einer Probe (11) im allgemeinen länglicher Form bestimmt sind, eine Trägerbrücke (6) für Belastungsmittel (13-15) der Probe (11), wobei die Belastungsmittel (13-15) eine Achse (15) umfassen, die zum Übertragen von Prüfbelastungen auf die Probe (11) rechtwinklig zu der Längsachse der Probe (11) bestimmt ist, und Mittel zur Messung der Belastungsbeanspruchung (26,27) sowie Mittel zur Messung des Fortschreitens einer Rißfront (32) umfaßt, wobei die Achse (15) an ihrem freien Ende mit einem austauschbaren Kopf (16) versehen ist, der so ausgebildet ist, daß er die Belastungsbeanspruchungen, die durch die Achse (15) ausgeübt werden, in Belastungsbeanspruchungen oder Biegebeanspruchungen umformen kann.

2. Vorrichtung nach Anspruch 1, **dadurch gekennzeichnet**, daß der Kopf (16) einen Bügel umfaßt, der dazu bestimmt ist, an der Probe (11) zwischen den Lagern (9,10) angebracht zu werden, wobei der Bügel (16) dergestalt ausgebildet ist, daß er eine Beanspruchung in zwei Richtungen auf die Probe (11) ausübt.

3. Vorrichtung nach Anspruch 2, **dadurch gekennzeichnet**, daß der Bügel (16) die Probe (11) über eine in Richtung der Längserstreckung der Probe (11) gemessene ausreichende Länge einschließt, um die Scherwirkung durch Zusammenwirken des Bügels (16) mit den Lagern (9,10) zu erreichen.

4. Vorrichtung nach einem der Ansprüche 1 bis 3, **dadurch gekennzeichnet**, daß sie Veränderungsmittel der Belastung (25,28) umfaßt, die es gestatten, gleichzeitig, aber in unabhängiger Weise, auf verschiedene Belastungsbeanspruchungen einzuwirken.

5. Vorrichtung nach einem der Ansprüche 1 bis 4, **dadurch gekennzeichnet**, daß sie Meßmittel (32,33) umfaßt, die es gestatten, die Reaktion der Probe (11) auszuwerten.

6. Vorrichtung nach einem der Ansprüche 1 bis 5, **dadurch gekennzeichnet**, daß der Sockel (2) eine Oberfläche (6) zeigt, auf der Trägerelemente (7,8) angeordnet sind, von denen jedes mit einem Stützelement (9,10) versehen ist, wobei jede Gesamtheit Trägerelement/Stützelement (7,9; 8,10) in der Weise ausgebildet ist, daß sie eine Übertragung parasitärer Wirkungen, die von einer unangebrachten Belastung des Rahmens (1) herrühren, zu der Probe (11) unterbinden kann.

7. Vorrichtung nach einem der Ansprüche 1 bis 6, **dadurch gekennzeichnet**, daß sie eine Klimaumhüllung (20) umfaßt, die dazu bestimmt ist, die Probe (11) aufzunehmen, um hierin die Versuche durchzuführen, wobei die Klimaumhüllung (20) mit einem dichten Durchgang (22) versehen ist, der für die Achse (15) bestimmt ist, und die an einen Klimatisierungsschrank (23) angeschlossen ist.

8. Vorrichtung nach einem der Ansprüche 1 bis 7, **dadurch gekennzeichnet**, daß sie mit einer programmierbaren Steuervorrichtung verbunden ist, die geeignet ist, verschiedene Belastungsmittel (13 bis 15), Klimatisierungsmittel (23) und Meßmittel (32,33) gemäß einer Folge von vorgegebenen Versuchsschritten zu steuern.

9. Verfahren zur Prüfung des Ermüdungsrißwachstums von Straßendeckenmaterialien sowohl durch Biegung als auch durch Scherung, umfassend die folgenden Schritte:

- Bilden einer Probe (11) im allgemeinen länglicher Form aus einem Material, das bestimmt ist, für eine Oberdecke einer Straße verwendet zu werden,
- Anreissen eines Risses auf einem Abschnitt der Oberfläche der Probe (11), wobei der Riß im wesentlichen quer bezüglich der Längsausdehnung der Probe (11) gerichtet ist,
- Anbringen der Probe (11) in einer Versuchsvorrichtung nach einem der Ansprüche 1 bis 8,
- Versehen der Probe (11) mit einem Sensor (32) des Fortschreitens der Rißfront,
- Verbinden des Sensors mit der Meßeinrichtung (26,27),
- Anbringen der Belastungsachse des Versuchsapparats an der Probe 11) mittels eines austauschbaren Kopfes (16), der es gestattet, die Belastungswirkungen, die durch die Achse ausgeübt werden, in Scherbeanspruchungen oder Biegebeanspruchungen zu transformieren..

- Programmieren der Steuereinrichtung mit den verschiedenen Versuchsparametern der Scherung oder der Biegung, die für den Versuchsablauf notwendig sind,

- Messen und Registrierung des Ablaufs der Rißfront und

- Bestimmen der wesentlichen Parameter des Straßendeckenmaterials auf der Grundlage von registrierten Meßergebnissen.

10. Verfahren nach Anspruch 9,
**dadurch gekennzeichnet**,
daß die Probe (11) die Form eines Parallelepipeds hat und daß der Riß in der Ebene angerissen ist, die dazu bestimmt ist, gegenüber der Oberfläche des Sockels des Rahmens (1), auf der die Stützelemente angebracht sind, orientiert zu sein, sobald die Probe (11) in dem Versuchsapparat angebracht ist.

11. Verfahren nach Anspruch 9 oder 10,
**dadurch gekennzeichnet**,
daß die Probe (11) als Einschichtenmaterial ausgebildet ist.

12. Verfahren nach Anspruch 9 oder 10,
**dadurch gekennzeichnet**,
daß die Probe (11) aus Mehrschichtenmaterial ausgebildet ist.

13. Verfahren nach einem der Ansprüche 9 bis 12,
**dadurch gekennzeichnet**,
daß die wichtigen Parameter die Parameter A und m des Gesetzes von Paris sind.

14. Verfahren nach einem der Ansprüche 9 bis 13,
**dadurch gekennzeichnet**,
daß der Sensor des Fortschreitens (26,27) durch wenigstens eine Schicht von gedruckten Widerstandsleitungen gebildet ist.

FIG.1

FIG.2

EP 0 603 029 B1